# EUROPÄISCHE PATENTSCHRIFT

(11) **EP 0 612 748 B1**
(45) Veröffentlichungstag und Bekanntmachung des Hinweises auf die Patenterteilung: **07.02.1996**
(21) Anmeldenummer: 94102710.4
(22) Anmeldetag: 23.02.1994
(51) Int. Cl.: C07D 493/04, C07C 51/265, B01J 21/06, B01J 23/22, B01J 27/185, B01J 27/198

(54) **Verfahren zur Herstellung von Pyromellitsäuredianhydrid (PMDA)**
Process for the preparation of pyromellitic dianhydride (PMDA)
Procédé pour la préparation de dianhydride pyromellitique (PMDA)

(30) Priorität: 25.02.1993 DE 4305817
(43) Veröffentlichungstag der Anmeldung: 31.08.1994
(73) Patentinhaber: Consortium für elektrochemische Industrie GmbH, D-81379 München (DE)
(72) Erfinder: Wagner, Werner, Dr., D-81541 München (DE); Müller, Frank, Dr., D-82266 Inning-Bachern (DE); Eberle, Hans-Jürgen, Dr., D-81477 München (DE); Grundei, Franz, D-85560 Ebersberg (DE)

(56) Entgegenhaltungen:
- EP-A- 0 046 397
- EP-A- 0 163 231
- EP-A- 0 330 195
- EP-A- 0 405 508
- AT-B- 169 330
- DE-A- 1 943 510
- DE-A- 3 730 747
- DE-A- 3 734 469
- DE-B- 1 468 824
- US-A- 4 719 311
- PATENT ABSTRACTS OF JAPAN vol. 4, no. 182 (C-35) (664) 16. Dezember 1980 & JP-A-55 122 787 (MITSUI TOATSU KAGAKU K.K.) 20. September 1980

## Beschreibung

Die Erfindung betrifft ein Verfahren zur Herstellung von Pyromellitsäuredianhydrid (PMDA) durch heterogen katalysierte Oxidation in der Gasphase mittels eines molekularen Sauerstoff enthaltenden Gases.

PMDA wird bisher großtechnisch hauptsächlich durch Flüssigphasenoxidation von 2,4,5-Trimethylbenzaldehyd mit Luftsauerstoff gewonnen; in einer Verfahrensweise, die der in der DE-A 1943510 (GB-A 1282775) beschriebenen Verfahrensweise zur Herstellung von Terephthalsäure aus p-Toluylaldehyd analog ist, wobei die so erhaltene Pyromellitsäure zu PMDA dehydratisiert wird. 2,4,5-Trimethylbenzaldehyd wird dabei durch Carbonylierung von 1,2,4-Trimethylbenzol (pseudo-Cumol) hergestellt (DE-A 2422197 = GB-A 1422308). Die Verwendung von Essigsäure als Lösungsmittel und Schwermetallsalze in Kombination mit einer Bromid-Quelle (Chem-Systems-Report: PERP 1987-T-4, 16-40) als Katalysatoren machen bei dieser Verfahrensweise den Einsatz von hochwertigen und somit sehr teuren Legierungen (Hastelloy C) für den Reaktor zwingend. Neben der batchweisen Fahrweise liegt ein weiterer Nachteil dieses Verfahrens darin, daß die durch Flüssigphasenoxidation gewonnene Pyromellitsäure in einem sehr energieintensiven Schritt (>200°C) zu PMDA dehydratisiert werden muß.

Ein weiteres Verfahren zur PMDA-Herstellung nach dem Prinzip der Flüssigphasenoxidation ist das Amoco-Verfahren (US-A 4719311). Unter Verwendung eines ähnlichen Katalysators (Co-Mn-Br) wird 1,2,4,5-Tetramethylbenzol (Durol) mit Luftsauerstoff zu Pyromellitsäure oxidiert, die dann ebenfalls erst zu PMDA dehydratisiert werden muß. Zu den beim obengenannten Verfahren beschriebenen Nachteilen kommt bei diesem Verfahren hinzu, daß Durol ca. fünfmal teurer ist als pseudo-Cumol.

Ein drittes PMDA-Verfahren arbeitet in der Gasphase. Analog der o-Xylol-Oxidation zu Phthalsäureanhydrid (PSA) wird an einem heterogenen Schalenkatalysator in einem Rohrbündelreaktor Durol direkt zu PMDA oxidiert. Als Katalysatorbestandteile nennt die US-A 4665200 V₂O₅, TiO₂, P₂O₅, Nb₂O₅, Sb₂O₃, K₂O und Cs₂O. Besondere Vorteile der Gasphasenoxidation sind die kontinuierliche Fahrweise und die problemlose Handhabbarkeit der Katalysatoren, wodurch beim Anlagenbau auf teure Materialien verzichtet werden kann. Prinzipiell ist es möglich, mit geringem Aufwand bereits bestehende PSA-Anlagen für die PMDA-Produktion umzurüsten. Die energieintensive Dehydratisierung des Flüssigphasenprozesses entfällt, da aus dem Reaktionsgas bereits das Anhydrid desublimiert wird. Durch geeignete Desublimationsverfahren (DE-A 3730747 = US-A 4867763) wird eine PMDA-Reinheit von 99 % erreicht.

Eine weitere Möglichkeit der PMDA-Gewinnung aus dem Reaktionsgas besteht in einer Gaswäsche mit einem wasserfreien Lösungsmittel, eine Technologie, die beispielsweise bei der Herstellung von Maleinsäureanhydrid Stand der Technik ist (SRI International, PEP-Report 46C, 1989). Weitere Beispiele für die Herstellung von PMDA über Gasphasenoxidation in Gegenwart von Vanadium- bzw. Titan-haltigen Katalysatoren sind in der EP-A 405508 und der EP-A 330195 beschrieben. Nachteilig bei der Gasphasenoxidation mit den bisher bekannten Verfahren ist die im Vergleich zu Flüssigphasenoxidations-Verfahren geringere Selektivität.

Für die Gasphasenoxidation sind neben 1,2,4,5-tetraalkylierten Benzolen als Ausgangsprodukte auch funktionell substituierte Benzolderivate beschrieben, die aus trisubstituierten Benzolen, z.B. pseudo-Cumol, hergestellt werden. Als funktionelle Gruppen beschrieben sind Chlormethyl und Alkoxymethyl (AT-PS 169 330). Aus ökologischen Gründen sind chlorhaltige Aromaten bedenklich, gerade bei so hohen Reaktionstemperaturen, wie das bei der Gasphasenoxidation der Fall ist. Alkoxymethylbenzole werden ebenfalls über eine Chlormethylierung hergestellt und sind aus dem gleichen Grund zu vermeiden.

Zusammengefaßt sind die gravierensten Nachteile der aus dem Stand der Technik bekannten Verfahrensweisen für die Verfahren der Flüssigphasenoxidation die teuren Reaktormaterialien aufgrund der korrosiven Katalysatoren, die langen Standzeiten durch den batchweisen Produktionsbetrieb, die energieaufwendige Dehydratisierung der Säure zum Anhydrid und für die Verfahren der Gasphasenoxidation deren teure Rohstoffbasis und geringe Selektivität.

Die der Erfindung zugrundeliegende Aufgabe war deshalb, ein Verfahren zu entwickeln, das die Vorteile der Flüssigphasenoxidation, nämlich die günstige Rohstoffbasis, mit den Vorteilen der Gasphasenoxidation, nämlich preiswerte Reaktormaterialien, kontinuierlicher Produktionsbetrieb, Umgehung der Dehydratisierung durch Desublimation des Anhydrids, miteinander verbindet und dabei mit möglichst hoher Selektivität zu dem gewünschten Produkt führt.

Es zeigte sich, daß gerade der Einsatz von alkylierten Benzaldehyden in der Gasphasenoxidation besonders vorteilhaft ist. Im Vergleich zur Durol-Oxidation läuft die Aldehyd-Oxidation wesentlich selektiver ab, das heißt die PMDA-Ausbeute steigt. Dies war nicht zu erwarten, denn aromatische Aldehyde gehen bei solch hohen Temperaturen wie sie in einem Gasphasenreaktionsrohr vorherrschen sehr leicht Decarbonylierungs- bzw. Zersetzungsreaktionen ein und sollten somit als Ausgangsprodukt für die Gasphasenoxidation zu PMDA ungeeignet sein. Ein weiterer wünschenswerter Effekt ist, daß die Bildung von Nebenprodukten, besonders Trimellitsäureanhydrid (TMSA), zurückgedrängt wird. Der Preisvorteil der pseudo-Cumol-Basis kann damit mit in Anspruch genommen werden, da analog des ersten Schrittes der Flüssigphasenoxidation Trimethylbenzaldehyd (TMBA) durch Carbonylierung relativ einfach aus dem billigen pseudo-Cumol hergestellt werden kann.

Gegenstand der Erfindung ist ein Verfahren zur Herstellung von Pyromellitsäuredianhydrid (PMDA) durch heterogen katalysierte Oxidation in der Gasphase mittels eines molekularen Sauerstoff enthaltenden Gases bei einer Temperatur von 250 bis 600°C, dadurch gekennzeichnet, daß mit C₁- bis C₃-Alkylgruppen 2,4,5-trialkylierte Benzaldehyde oder Gemische aus mit C₁- bis C₃-Alkylgruppen 2,4,5-trialkylierten Benzaldehyden und mit C₁- bis C₃-Alkylgruppen 1,2,4,5-tetraalkylierten Benzolen in einem Verhältnis von 10 : 1 bis 1 : 10, in Gegenwart eines Kataly-sators oxidiert werden, welcher als Aktivkomponenten 5 bis 95 Gew% eines oder mehrerer Übergangsmetalloxide der 4. Nebengruppe des PSE, 1 bis 50 Gew% eines oder mehrerer Übergangsmetalloxide der 5. Nebengruppe des PSE, 0 bis 10 Gew% eines oder mehrerer Oxide von Elementen der 1. Hauptgruppe des PSE und/oder 0 bis 50 Gew% eines oder mehrerer Oxide von Elementen der 3., 4. und 5. Hauptgruppe des PSE und von Elementen der 6. und 7. Nebengruppe des PSE enthält, wobei die Angaben in Gew% jeweils auf das Gesamtgewicht der Aktivkomponenten bezogen sind und sich auf 100 Gew% aufaddieren.

Die Gewinnung von PMDA erfolgt durch katalytische Gasphasenoxidation ausgehend von 2,4,5-trialkylierten Benzaldehyden, in der die Alkylgruppen Methyl-, Ethyl-, Propyl- oder Isopropylreste sein können, oder ausgehend von einem Gemisch aus 2,4,5-trialkylierten Benzaldehyden, in der die Alkylgruppen Methyl-, Ethyl-, Propyl- oder Isopropylreste sein können, und 1,2,4,5-tetraalkylierten Benzolen, in der die Alkylgruppen ebenfalls Methyl-, Ethyl-, Propyl- oder Isopropylreste sein können. Werden diese Gemische eingesetzt, beträgt das Verhältnis von 2,4,5-trialkylierten Benzaldehyden zu 1,2,4,5-tetraalkylierten Benzolen vorzugsweise 10 : 1 bis 1 : 10. Vorzugsweise wird 2,4,5-Trimethylbenzaldehyd oder ein Gemisch aus 2,4,5-Trimethylbenzaldehyd und 1,2,4,5-Tetramethylbenzol (Durol) eingesetzt. Besonders bevorzugt wird einzig 2,4,5-Trimethylbenzaldehyd als Edukt eingesetzt.

Vorzugsweise enthält der Katalysator als Aktivkomponenten 10 bis 90 Gew% eines Oxids von Titan und/oder Zirkonium, 5 bis 35 Gew% eines Oxids von Vanadium und/oder Niob sowie 0 bis 5 Gew% eines oder mehrerer Oxide aus der Gruppe der Oxidverbindungen von Kalium, Rubidium, Cäsium und/oder 0.1 bis 10 Gew% eines oder mehrerer Oxide aus der Gruppe Phosphor, Antimon, Wismuth, Chrom, Molybdän, Wolfram, Mangan, wobei die Angaben in Gewichtsprozent jeweils auf das Gesamtgewicht der Aktivkomponenten bezogen sind.

Besonders bevorzugt werden Zusammensetzungen mit Titandioxid als Oxid von übergangsmetallen der 4. Nebengruppe des PSE, Vanadiumpentoxid als Oxid von übergangsmetallen der 5. Nebengruppe des PSE, welche mit Phosphorpentoxid, gegebenenfalls zusammen mit Sb₂O₃ und/oder Cs₂O, dotiert sind. Am meisten bevorzugt sind dabei Zusammensetzungen, welche Titandioxid in der Anatas-Modifikation mit einer BET-Oberfläche von 5 bis 200 m²/g enthalten.

Der Katalysator kann als Vollkontakt (Preßlinge, Extrudate, Granulate) oder in Form von Kontakten mit schalenartigem Aufbau eingesetzt werden. Die Form richtet sich dabei nach dem Verfahren der Gasphasenoxidation. So werden beispielsweise im Fließbettverfahren Katalysatoren in Granulatform und im Festbettverfahren Preßlinge oder mit aktiver Masse beschichtete Ringe oder Kugeln (Schalenkontakte) eingesetzt.

Vorzugsweise wird nach dem Festbettverfahren gearbeitet; dabei befindet sich die katalytisch aktive Masse auf inerten Trägermaterialien. Der Anteil der aktiven Masse beläuft sich, bezogen auf die Gesamtmasse, also der Massen von Trägerkörper und aktiver Masse, auf 1 bis 30 Gew%, vorzugsweise 2 bis 15 Gew%. Im Prinzip können die Träger beliebige Gestalt und Oberflächenstruktur besitzen. Bevorzugt werden jedoch regelmäßig geformte, mechanisch stabile Körper wie Kugeln, Ringe, Halbringe, Zylinder, Sättel, mit glatter, porenfreier Oberfläche. Die Größe der Trägerkörper wird vorwiegend von der Dimension, vor allem vom inneren Durchmesser der Reaktionsrohre bestimmt, wenn der Katalysator in Röhren- bzw. Rohrbündelreaktoren zur Anwendung kommt. Der Trägerdurchmesser sollte dann zwischen 1/2 und 1/10 des Innendurchmessers betragen. Als Materialien eignen sich beispielsweise Steatit, Duranit, Siliciumcarbid, Steingut, Porzellan, Siliciumdioxid, Silicate, Aluminiumoxid, Aluminate oder Mischungen aus diesen Stoffen. Vorzugsweise werden Kugeln oder Ringe aus Steatit eingesetzt.

Das Aufbringen der Aktivkomponenten auf die inerten Träger kann auf an sich bekannte Art und Weise erfolgen. So können die Träger mit einer wäßrigen Aufschlämmung der Mischung oder aber der einzelnen Komponenten in einem Drehrohrofen bei 200 - 300°C beschichtet werden. Die Aktivkomponenten können in Form der Oxide oder in Form von Verbindungen aufgetragen werden, welche sich unter den Bedingungen der Gasphasenoxidation oder bei einem vorgelagerten Temperschritt in die Oxide umwandeln. Trägerkatalysatoren mit sehr gut haftenden Überzügen erhält man, indem eine wäßrige Suspension, welche die Mischung oder die einzelnen Komponenten sowie einen organischen Binder enthält, auf die Trägerkörper aufgebracht wird. Derartige Verfahren zur Beschichtung von Trägerkatalysatoren sind beispielsweise aus der DE-B 2106796 (US-A 3799886) bekannt.

Bei der erfindungsgemäßen Verfahrensweise werden die Edukte zusammen mit einem sauerstoffhaltigen Gas, in Gegenwart des beschriebenen Oxidationskatalysators, vorzugsweise in Festbettreaktoren umgesetzt. Übliche Festbettreaktoren sind beispielsweise Reaktionsrohre, welche zu einem Rohrbündelreaktor zusammengefaßt und von einem Wärmeaustauschmedium umgeben sind. Die Reaktionsrohre sind vertikal angeordnet und werden vom Reaktionsgemisch von oben her durchströmt; sie bestehen aus einem gegenüber Wärmaustauschermedium, Katalysator, Edukten und Produkten inertem Material, im allgemeinen einem geeigneten Stahl, und besitzen eine Länge von 2000 bis 4000 mm, vorzugsweise 2500 bis 3500 mm, einen Innendurchmesser von 10 bis 30 mm, vorzugsweise 18 bis 26 mm, und eine Wandstärke von 1 bis 4 mm. Als Wärmeaustauschermedien haben sich in der industriellen Praxis eutektische Salzgemische bewährt, beispielsweise eine chloridfreie Schmelze aus Kaliumnitrat und Natriumnitrit.

Der Katalysator wird von oben in die Reaktionsrohre eingefüllt und durch in der Nähe der unteren Rohrenden angebrachte Halterungen fixiert. Die Füllhöhe kann zwischen 900 und 3300 mm betragen. Die Reaktionsrohre können gegebenenfalls schichtweise mit Trägerkörpern unterschiedlicher Gestalt und Dimension sowie unterschiedlicher Konzentration und Zusammensetzung der Aktivkomponenten befüllt werden.

In dem erfindungsgemäßen Verfahren wird das Reaktionsgas enthaltend 2,4,5-trialkylierten Benzaldehyd, gegebenenfalls im Gemisch mit 1,2,4,5-tetraalkyliertem Benzol, mit einem sauerstoffhaltigen Gas, vorzugsweise Luft, über den Katalysator geleitet. Vorzugsweise betragen die Raumgeschwindigkeiten 800 bis 8000 h⁻¹, besonders bevorzugt 1000 bis 6000 h⁻¹. Dabei beträgt das Mischungsverhältnis 10 bis 100 g Edukt/Nm³, vorzugsweise 10 bis 40 g Edukt/Nm³. Die Reaktionstemperatur beträgt 250 bis 600°C, vorzugsweise 300 bis 500°C.

Nach der Umsetzung wird das gebildete Pyromellitsäuredianhydrid (PMDA) in an sich bekannter Weise durch Desublimation in einem nachgeschalteten Abscheider bei 40 bis 80°C (DE-A 3730747 = US-A 4867763) oder durch entsprechende Gaswäsche mit einem geeigneten Lösungsmittel aus dem Reaktionsgas gewonnen.

Das mit dem erfindungsgemäßen Verfahren zugängliche Pyromelittsäuredianhydrid wird als Ausgangsmaterial (Comonomer) zur Herstellung von hochtemperaturbeständigen Polymeren, als Härter für Epoxidharze und als Ausgangsmaterial für Weichmacherkomponenten verwendet.

Die nachfolgenden Beispiele dienen der weiteren Erläuterung der Erfindung.

### Katalysatorpräparation:

55 g TiO₂ (Anatas), 7 g V₂O₅ und 3.5 g (NH₄)₂HPO₄ wurden in 400 ml entionisiertem Wasser suspendiert und 18 Stunden gerührt, um eine homogene Verteilung zu erzielen. Vor dem Auftragen der Mischung auf 1000 g 8 mm-Steatitkugeln wurden der Suspension 20 g Copolymeres aus Vinylacetat und Vinyllaurat in Form einer 50 %-igen wäßrigen Dispersion zugegeben. Anschließend wurde die Suspension unter Verdampfen des Wassers auf den Träger aufgebracht. Nach einem Temperschritt von 4 Stunden bei 410°C und einem Luftstrom von 0.5 Nm³/h hatte die katalytisch aktive Masse eine Oberfläche von 95 m²/g (gemessen nach BET).

Alle folgenden Beispiele wurden in einem technischen Maßstäben nachempfundenen Reaktionsrohr durchgeführt. Die Länge des Reaktorrohres betrug 3.3 m (Füllhöhe 2.8 m, entsprechend einer Katalysatormenge von 1730 g), dessen Durchmesser 25 mm. Der Reaktor wurde mit einem umgewälzten Salzbad (eutektische, chloridfreie Salzschmelze aus Kaliumnitrat und Natriumnitrit) temperiert. Die eingespeiste Luftmenge lag bei 4 Nm³/h. Das Mischungsverhältnis Edukt/Luft betrug 12 bis 35 g/Nm³ Luft. Die Reinheit des 2,4,5-Trimethylbenzalde-hyds betrug zwischen 95 und 98 Gew%. Die Reinheit des 1,2,4,5-Tetramethylbenzols (Durol) in den Vergleichsbeispielen betrug von 97 bis 99 Gew%.

Die Reaktionsbedingungen und Ausbeuten der zwei Beispiele und der zwei Vergleichsbeispiele zeigt die nachfolgende Ta-belle.

**TABELLE**

| | Beispiel 1 | Beispiel 2 | Vergl.-beispiel 1 | Vergl.-beispiel 2 |
|---|---|---|---|---|
| Edukt | TMBA | TMBA | Durol | Durol |
| SBT [°C] | 375 | 380 | 370 | 375 |
| PMDA [Gew%*] | 83 | 90 | 75 | 75 |
| TMSA [Gew%**] | 0.8 | 0.7 | 5 | 4 |

| | | | | |
|---|---|---|---|---|
| * = bezogen auf 100%-iges Ausgangsprodukt | | | | |
| ** = bezogen auf PMDA | | | | |
| SBT = Salzbadtemperatur TMBA = 2,4,5-Trimethylbenzaldehyd TMSA = Trimellitsäureanhydrid | | | | |

Um den TMSA-Gehalt des abgeschiedenen PMDA analysieren zu können, wurde das Reaktionsprodukt mit einem H₂SO₄/CH₃OH-Gemisch (1:3 Vol%) in die Methylester überführt und der TMSA-Gehalt anschließend gaschromatographisch bestimmt.

## Patentansprüche

1. Verfahren zur Herstellung von Pyromellitsäuredianhydrid (PMDA) durch heterogen katalysierte Oxidation in der Gasphase mittels eines molekularen Sauerstoff enthaltenden Gases bei einer Temperatur von 250 bis 600°C, dadurch gekennzeichnet, daß mit C₁- bis C₃-Alkylgruppen 2,4,5-trialkylierte Benzaldehyde oder Gemische aus mit C₁- bis C₃-Alkylgruppen 2,4,5-trialkylierten Benzaldehyden und mit C₁- bis C₃-Alkylgruppen 1,2,4,5-tetraalkylierten Benzolen in einem Verhältnis von 10 : 1 bis 1 : 10, in Gegenwart eines Katalysators oxidiert werden, welcher als Aktivkomponenten 5 bis 95 Gew% eines oder mehrerer Übergangsmetalloxide der 4. Nebengruppe des PSE, 1 bis 50 Gew% eines oder mehrerer Übergangsmetalloxide der 5. Nebengruppe des PSE, 0 bis 10 Gew% eines oder mehrerer Oxide von Elementen der 1. Hauptgruppe des PSE und/oder 0 bis 50 Gew% eines oder mehrerer Oxide von Elementen der 3., 4. und 5. Hauptgruppe des PSE und von Elementen der 6. und 7. Nebengruppe des PSE enthält, wobei die Angaben in Gew% jeweils auf das Gesamtgewicht der Aktivkomponenten bezogen sind und sich auf 100 Gew% aufaddieren.

2. Verfahren nach Anspruch 1, dadurch gekennzeichnet, daß 2,4,5-Trimethylbenzaldehyd oder ein Gemisch aus 2,4,5-Trimethylbenzaldehyd und 1,2,4,5-Tetramethylbenzol eingesetzt wird.

3. Verfahren nach Anspruch 1 bis 2, dadurch gekennzeichnet, daß die Oxidation in einem Festbettreaktor mit Luft bei Raumgeschwindigkeiten von 800 bis 8000 h⁻¹, einem Mischungsverhältnis 10 bis 100 g Edukt/Nm³ und einer Reaktionstemperatur von 250 bis 600°C durchgeführt wird und das gebildete Pyromellitsäuredianhydrid (PMDA) durch Desublimation in einem nachgeschalteten Abscheider oder durch eine entsprechende Gaswäsche mit einem geeigneten Lösungsmittel aus dem Reaktionsgas gewonnen wird.

4. Verfahren nach Anspruch 1 bis 3, dadurch gekennzeichnet, daß als Aktivkomponenten 5 bis 95 Gew% eines oder mehrerer Übergangsmetalloxide der 4. Nebengruppe des PSE, 1 bis 50 Gew% eines oder mehrerer Übergangsmetalloxide der 5. Nebengruppe des PSE, 0 bis 10 Gew% eines oder mehrerer Oxide von Elementen der 1. Hauptgruppe des PSE und/oder 0 bis 50 Gew% eines oder mehrerer Oxide von Elementen der 3., 4. und 5. Hauptgruppe des PSE und von Elementen der 6. und 7. Nebengruppe des PSE enthalten sind.

5. Verfahren nach Anspruch 4, dadurch gekennzeichnet, daß als Aktivkomponenten 10 bis 90 Gew% eines Oxids von Titan und/oder Zirkonium, 5 bis 35 Gew% eines Oxids von Vanadium und/oder Niob sowie 0 bis 5 Gew% eines oder mehrerer Oxide aus der Gruppe der Oxidverbindungen von Kalium, Rubidium, Cäsium und/oder 0.1 bis 10 Gew% eines oder mehrerer Oxide aus der Gruppe Phosphor, Antimon, Wismuth, Chrom, Molybdän, Wolfram, Mangan, wobei die Angaben in Gewichtsprozent jeweils auf das Gesamtgewicht der Aktivkomponenten bezogen sind, enthalten sind.

6. Verfahren nach Anspruch 1 bis 5, dadurch gekennzeichnet, daß der Anteil der aktiven Masse, bezogen auf die Massen von Trägerkörper und aktiver Masse, 1 bis 30 Gew% beträgt und als Träger Kugeln oder Ringe verwendet werden.

7. Verfahren nach Anspruch 1 bis 6, dadurch gekennzeichnet, daß als Aktivkomponenten Titandioxid in der Anatas-Modifikation mit einer BET-Oberfläche von 5 bis 200 m²/g, Vanadiumpentoxid und Phosphorpentoxid, gegebenenfalls zusammen mit Sb₂O₃ und/oder Cs₂O, enthalten sind.

## Claims

1. Process for preparing pyromellitic dianhydride (PMDA) by heterogeneously catalysed oxidation in the gas phase by means of a gas containing molecular oxygen at a temperature of from 250 to 600°C, characterized in that benzaldehydes which are 2,4,5-trialkylated by C₁- to C₃-alkyl groups or mixtures of benzaldehydes which are 2,4,5-trialkylated by C₁- to C₃-alkyl groups and benzenes which are 1,2,4,5-tetraalkylated by C₁- to C₃-alkyl groups in a ratio of from 10:1 to 1:10 are oxidized in the presence of a catalyst which contains as active components from 5 to 95 % by weight of one or more transition-metal oxides of sub-group IV of the Periodic Table of the Elements, from 1 to 50 % by weight of one or more transition-metal oxides of sub-group V of the Periodic Table of the Elements, from 0 to 10 % by weight of one or more oxides of elements of main group I of the Periodic Table of the Elements and/or from 0 to 50 % by weight of one or more oxides of elements of main groups III, IV and V of the Periodic Table of the Elements and of elements of sub-groups VI and VII of the Periodic Table of the Elements, where the indicated percentages by weight are based in each case on the total weight of the active components and add to 100 % by weight.

2. Process according to Claim 1, characterized in that 2,4,5-trimethylbenzaldehyde or a mixture of 2,4,5-trimethylbenzaldehyde and 1,2,4,5-tetramethylbenzene is used.

3. Process according to Claim 1 or 2, characterized in that the oxidation is carried out in a fixed-bed reactor with air at space velocities of from 800 to 8000 h⁻¹, a mixing ratio of from 10 to 100 g of starting material/Nm³ and a reaction temperature of from 250 to 600°C, and the pyromellitic dianhydride (PMDA) is isolated from the reaction gas by desublimation in a downstream separator or by corresponding gas scrubbing with a suitable solvent.

4. Process according to any of Claims 1 to 3, characterized in that the active components contained are from 5 to 95 % by weight of one or more transition-metal oxides of sub-group IV of the Periodic Table of the Elements, from 1 to 50 % by weight of one or more transition-metal oxides of sub-group V of the Periodic Table of the Elements, from 0 to 10 % by weight of one or more oxides of elements of main group I of the Periodic Table of the Elements and/or from 0 to 50 % by weight of one or more oxides of elements of main groups III, IV and V of the Periodic Table of the Elements and of elements of sub-groups VI and VII of the Periodic Table of the Elements.

5. Process according to Claim 4, characterized in that the active components contained are from 10 to 90 % by weight of an oxide of titanium and/or zirconium, from 5 to 35 % by weight of an oxide of vanadium and/or niobium and also from 0 to 5 % by weight of one or more oxides from the group consisting of oxide compounds of potassium, rubidium, caesium and/or from 0.1 to 10 % by weight of one or more oxides from the group consisting of phosphorus, antimony, bismuth, chromium, molybdenum, tungsten, manganese, where the indicated percentages by weight are based in each case on the total weight of the active components.

6. Process according to any of Claims 1 to 5, characterized in that the proportion of the active composition, based on the masses of support bodies and active composition, is from 1 to 30 % by weight and the supports used are beads or rings.

7. Process according to any of Claims 1 to 6, characterized in that the active components contained are titanium dioxide in the anatase form having a BET surface area of from 5 to 200 m²/g, vanadium pentoxide and phosphorus pentoxide which may also contain Sb₂O₃ and/or Cs₂O.

## Revendications

1. Procédé de préparation de dianhydride pyromellitique (PMDA) par oxydation à catalyse hétérogène en phase gazeuse, au moyen d'un gaz contenant de l'oxygène moléculaire à une température de 250 à 600°C, caractérisé en ce que du benzaldéhyde 2,4,5-trialcoylé par des groupes alcoyle en C₁ à C₃ ou des mélanges de benzaldéhyde 2,4,5-trialcoylé par des groupes alcoyle en C₁ à C₃ et de benzène 1,2,4,5-tétraalcoylé par des groupes alcoyle en C₁ à C₃ en un rapport de 10:1 à 1:10 sont oxydés en présence d'un catalyseur qui contient comme composants actifs, 5 à 95% en poids d'un ou plusieurs oxydes de métal de transition du groupe 4B du tableau périodique des éléments, 1 à 50% en poids d'un ou plusieurs oxydes de métal de transition du groupe 5B du tableau périodique des éléments, 0 à 10% en poids d'un ou de plusieurs oxydes des éléments du groupe 1A et/ou 0 à 50% en poids d'un ou de plusieurs oxydes des éléments des groupes 3A, 4A et 5A du tableau périodique des éléments et des éléments des groupes 6B et 7B du tableau périodique des éléments, où les données en % en poids sont rapportées au poids total des composants actifs et s'additionnent pour faire 100% en poids.

2. Procédé suivant la revendication 1, caractérisé en ce que l'on utilise le 2,4,5-triméthylbenzaldéhyde ou un mélange de 2,4,5-triméthylbenzaldéhyde et de 1,2,4,5-tétraméthylbenzène.

3. Procédé suivant les revendications 1 et 2, caractérisé en ce que l'oxydation est réalisée dans un réacteur à lit fixe avec de l'air à une vitesse d'écoulement de 800 à 8000 h⁻¹, un rapport de mélange de 10 à 100 g déduit par Nm³ et une température de réaction de 250 à 600°C et le dianhydride pyromellitique (PMDA) formé est obtenu par sublimation dans un séparateur postérieur ou par lavage gazeux correspondant du gaz réactionnel par un solvant approprié.

4. Procédé suivant les revendications 1 à 3, caractérisé en ce que les composants actifs comprennent 5 à 95% en poids d'un ou plusieurs oxydes de métal de transition du groupe 4B du tableau périodique des éléments, 1 à 50% en poids d'un ou plusieurs oxydes de métal de transition du groupe 5B du tableau périodique des éléments, 0 à 10% en poids d'un ou de plusieurs oxydes des éléments du groupe 1A et/ou 0 à 50% en poids d'un ou de plusieurs oxydes des éléments des groupes 3A, 4A et 5A du tableau périodique des éléments et des éléments des groupes 6B et 7B du tableau périodique des éléments.

5. Procédé suivant la revendication 4, caractérisé en ce que les composants actifs comprennent 10 à 90% en poids d'un oxyde du titane et/ou du zirconium, 5 à 35% en poids d'un oxyde du vanadium et/ou du niobium ainsi que 0 à 5% en poids d'un ou plusieurs oxydes choisis dans le groupe des composés oxydés du potassium, rubidium, césium et/ou 0,1 à 10% en poids d'un ou plusieurs oxydes du groupe phosphore, antimoine, bismuth, chrome, molybdène, tungstène, manganèse, où les quantités en pour-cent en poids sont rapportées au poids total de composants actifs.

6. Procédé suivant les revendications 1 à 5, caractérisé en ce que la composante de masse active, rapportée à la masse de corps support et de masse active, atteint 1 à 30% en poids et en ce que l'on utilise comme support, des billes ou anneaux.

7. Procédé suivant les revendications 1 à 6, caractérisé en ce que les composants actifs comprennent du dioxyde de titane sous forme anatase avec une surface BET de 5 à 200 m²/g, du pentoxyde de vanadium et du pentoxyde de phosphore, facultativement avec du Sb₂O₃ et/ou du Cs₂O.
